# EUROPEAN PATENT APPLICATION

(11) **EP 4 129 180 A1**
(43) Date of publication of application: **08.02.2023**
(21) Application number: 22188968.6
(22) Date of filing: 05.08.2022
(51) Int. Cl.: A61B 5/367

(54) **GRAPHICAL USER INTERFACE TEMPLATE FOR REDUCING SETUP TIME OF ELECTROPHYSIOLOGICAL PROCEDURES**

(30) Priority: 06.08.2021 US 202117395531
(71) Applicant: Biosense Webster (Israel) Ltd, Yokneam, 2066717 (IL)
(72) Inventor: GLINER, Vadim, 2066717 Yokneam (IL)
(74) Representative: Carpmaels & Ransford LLP

(57) **Abstract**

A method includes, inserting a catheter into a cavity of a patient organ for performing a medical procedure, the cavity is of a given cavity type. A partial anatomical mapping of the cavity is performed by visiting one or more anatomical points on a surface of the cavity, using the catheter. Based on the partial anatomical mapping, a Graphical User Interface (GUI) template is selected, which is specified for applying the medical procedure to the given cavity type. The selected GUI template is presented to a user for performing the medical procedure in the cavity.

## Description

### FIELD OF THE INVENTION

The present invention relates generally to medical systems, and particularly to methods and systems for using a graphical user interface template for reducing the setup time of electrophysiological procedures.

### BACKGROUND OF THE INVENTION

Some physicians that perform medical procedures, such as electro-anatomical mapping and/or tissue ablation procedures performed in a patient heart, may use one or more templates for automating the medical process and for improving the quality of the procedure.

For example, U.S. Patent Application Publication No. 2015/0282729 describes determination and/or representation of physiological information relating to a heart surface using various types of templates.

PCT International Publication WO 2021/084476 describes mapping electrical activity in heart tissue to guide the placement of devices to intervene in (e.g., treat) structural heart disease. In some exemplary embodiments, the intervention comprises placement of an implantable device, and/or positioning of a therapeutic device used to remove and/or remodel tissue. In some exemplary embodiments, electrical activity mapping is performed along with spatial mapping of a body cavity. In some exemplary embodiments, the intervention device position is compared to the measured positions of anatomical structures critical to heart electrical function to assess and/or prevent complications due to the device damaging heart electrical function.

### SUMMARY OF THE INVENTION

An embodiment of the present invention that is described herein provides a method including, inserting a catheter into a cavity of a patient organ for performing a medical procedure, the cavity is of a given cavity type. A partial anatomical mapping of the cavity is performed by visiting one or more anatomical points on a surface of the cavity, using the catheter. Based on the partial anatomical mapping, a Graphical User Interface (GUI) template is selected, which is specified for applying the medical procedure to the given cavity type. The selected GUI template is presented to a user for performing the medical procedure in the cavity.

In some embodiments, selecting the GUI template includes choosing the GUI templates from among multiple GUI templates specified for multiple respective cavity types. In other embodiments, selecting the GUI template includes applying, to the partial anatomical mapping, a neural network that is pre-trained using one or more other anatomical mappings of one or more cavities. In yet other embodiments, performing the partial anatomical mapping includes producing a triangular mesh of the visited anatomical points, and selecting the GUI template includes applying the pre-trained neural network to the triangular mesh.

In an embodiment, a first number of the visited anatomical points in the partial anatomical mapping is smaller than a second number of anatomical points used for training the neural network. In another embodiment, at least one of the one or more cavities is mapped in another patient. In yet another embodiments, the patient organ includes a patient heart, and the medical procedure includes an ablation of tissue of the patient heart.

In some embodiments, the patient organ includes a patient heart, and the medical procedure includes an electro-anatomical mapping of tissue of the patient heart. In other embodiments, selecting the GUI template includes identifying the given cavity type based on the partial anatomical mapping, and selecting the GUI template based on the identified cavity type. In yet other embodiments, selecting the GUI template includes selecting the GUI template based on the identified cavity type and on a type of the medical procedure.

There is additionally provided, in accordance with an embodiment of the present invention, a system including a processor and an output device. The processor is configured to: (i) receive, from a catheter inserted into a patient organ having a cavity of a given cavity type and visiting one or more anatomical points on a surface of the cavity, a partial anatomical mapping of the cavity, and (ii) select, based the partial anatomical mapping, a Graphical User Interface (GUI) template that is specified for applying the medical procedure to the given cavity type. The output device is configured to present the selected GUI template to a user for performing the medical procedure in the cavity.

### BRIEF DESCRIPTION OF THE DRAWINGS

The present invention will be more fully understood from the following detailed description of the embodiments thereof, taken together with the drawings in which:
Fig. 1 is a schematic, pictorial illustration of a catheter-based tracking and ablation system, in accordance with an exemplary embodiment of the present invention;
Fig. 2 is a flow chart that schematically illustrates a method for reducing the setup time of an electrophysiological procedure, in accordance with an exemplary embodiment of the present invention; and
Fig. 3 is a schematic block diagram illustrating a structure of a neural network applied to a partial anatomical mapping of a heart cavity for reducing the setup time of an electrophysiological procedure, in accordance with an exemplary embodiment of the present invention.

### DETAILED DESCRIPTION OF EMBODIMENTS

### OVERVIEW

Some medical procedures, such as electrophysiological (EP) procedures (e.g., electro-anatomical (EA) mapping and/or ablation of tissue) in a patient heart, typically require detailed set up operations for performing the procedure. For example, in performing an ablation procedure, a physician inserts a catheter into a cavity, e.g., a left atrium (LA), of the heart for performing the ablation in the LA. In some cases the procedure may comprise: (i) an anatomical mapping and an EA mapping of the LA, and subsequently, (ii) tissue ablation, which is planned and performed based on the EA mapping. The operations described above require set up operations that may consume time and, undesirably, prolong the duration of the procedure. For example, the setup operations may comprise producing a three-dimensional (3D) map of the LA, selection and analysis of EP signals acquired during the EA mapping, and producing a suitable graphical user interface (GUI) for performing the procedure.

Embodiments of the present invention that are described hereinbelow provide improved techniques for reducing the setup time of medical procedures by automating some steps of the setup. More specifically, reducing the setup time of EP procedures, such as EA mapping and/or ablation of tissue carried out in a selected cavity of the patient heart.

In some exemplary embodiments, after selecting the procedure type and heart chamber, e.g., ablation of tissue in the left atrium of the patient heart, a physician inserts a catheter having electrodes into the LA. Subsequently, the physician moves the catheter for visiting some anatomical points on a surface of the LA, and uses the electrodes for acquiring the anatomical points and their respective positions (e.g., using a suitable position tracking system).

In some exemplary embodiments, a system for performing EP procedures comprises a processor, which is configured to perform a partial anatomical mapping of the LA using the acquired anatomical points received from the catheter. In the present example, the processor produces a triangular mesh of the visited and acquired anatomical points. The processor is further configured to apply to the triangular mesh, a neural network (NN), such as a convolutional neural network (CNN) that may or may not be combined with a recurrent neural network (RNN), which is trained before conducting the EP procedure. The NN is pre-trained using other anatomical mappings that were previously stored in a memory of the system. For example, the other anatomical mappings may comprise full anatomical mappings of the heart chambers (including left atriums) of other patients.

In some exemplary embodiments, the processor is configured to produce or to receive, based on the data produced in the NN training, a set of templates for each EP procedure applied to each selected cavity (e.g., chamber) of the patient heart. In a simplified example, four templates are produced for performing EA mapping in the four chambers of the patient heart (left and right atriums and left and right ventricles), and an additional four templates are produced for performing tissue ablation in the aforementioned four chambers. Note that in some cases multiple templates may be produced for performing a given procedure in at least one of the chambers. For example, multiple ablation sites in different respective areas of a given chamber, may require multiple templates.

In some exemplary embodiments, after receiving sufficient anatomical points, the processor is configured to identify the LA of the patient heart based on the partial anatomical mapping. Moreover, the processor is configured to select, based the partial anatomical mapping, a Graphical User Interface (GUI) template that is specified for applying the medical procedure (e.g., tissue ablation) to the chamber in question (e.g., LA) of the patient heart.

In some exemplary embodiments, the system comprises an output device, such as a display, which is configured to present the selected GUI template (and LA map) to a user (the physician), for performing the tissue ablation in the LA.

In some cases the physician may decide to carry out an additional procedure in another chamber of the patient heart. For example, an EA mapping in the right atrium. In some exemplary embodiments, the physician inserts the catheter to the right atrium for performing a partial mapping as described above, and the processor is configured to select and present a corresponding template to the physician, using the techniques described in detail above.

The disclosed techniques improve the quality of medical procedures, such as but not limited to electrophysiological procedures, and reduce the cycle time thereof by automating at least some steps in the process of the procedure.

### SYSTEM DESCRIPTION

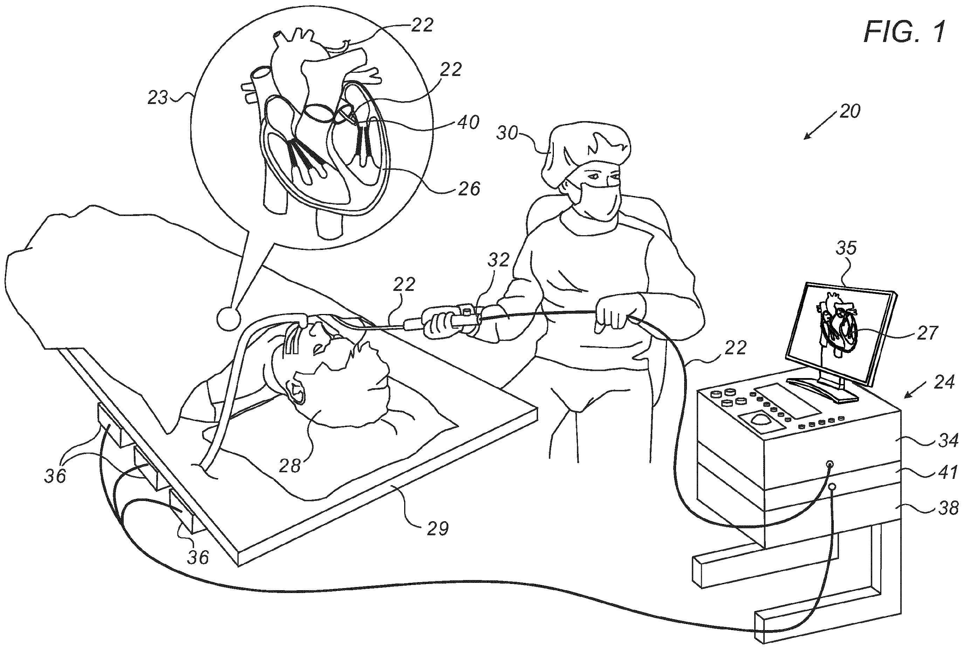

Fig. 1 is a schematic, pictorial illustration of a catheter-based tracking and ablation system 20, in accordance with an exemplary embodiment of the present invention.

In some exemplary embodiments, system 20 comprises a catheter 22, in the present example a cardiac catheter, and a control console 24. In the exemplary embodiment described herein, catheter 22 may be used for any suitable therapeutic and/or diagnostic purposes in a heart 26, such as electro-anatomical (EA) mapping and/or ablation of tissue using techniques described below.

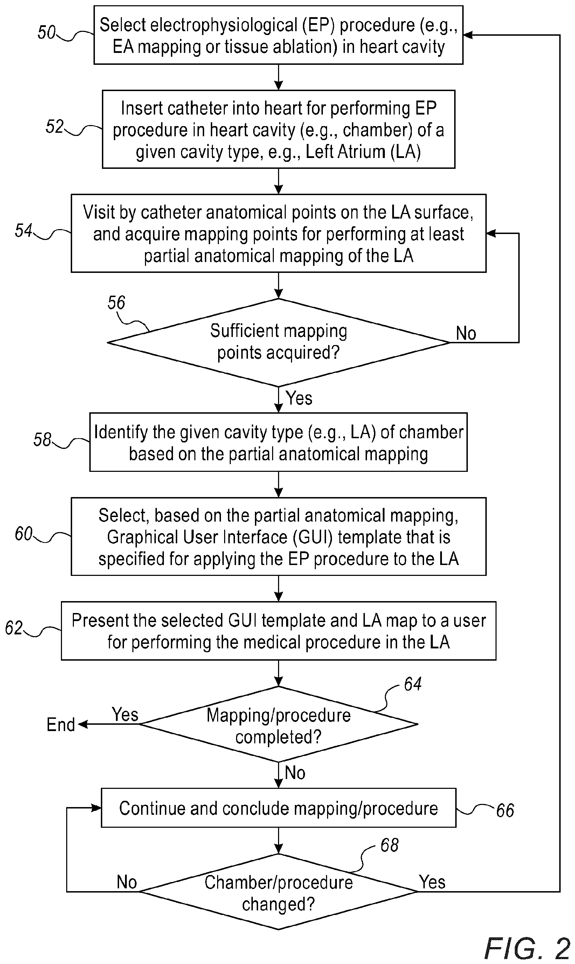

In some exemplary embodiments, console 24 comprises a processor 34, typically a general-purpose computer, with suitable front end and interface circuits 38 for receiving signals via catheter 22 and for controlling the other components of system 20 described herein. Console 24 further comprises an output device, in the present example a user display 35, which is configured to receive from processor 34 a map 27 of heart 26, and to display map 27 and additional components of a graphical user interface (GUI) based on templates described with respect to Fig. 2 below.

In some exemplary embodiments, map 27 may comprise any suitable type of a three-dimensional (3D) anatomical map produced using any suitable technique. For example, the anatomical map may be produced using an anatomical image produced by a suitable medical imaging system, or performing a fast anatomical mapping (FAM) techniques using the CARTO^{™} system, produced by Biosense Webster Inc. (Irvine, Calif.), or using any other suitable technique, or using any suitable combination of the above.

Reference is now made to an inset 23. In some exemplary embodiments, prior to performing an ablation procedure, a physician 30 inserts catheter 22 through the vasculature system of a patient 28 lying on a table 29, so as to perform an electro-anatomical mapping of tissue in question of heart 26.

In some exemplary embodiments, catheter 22 comprises a distal-end assembly 40 having multiple sensing electrodes (not shown). For example, distal-end assembly 40 may comprise: (i) a basket catheter having multiple splines, each spline having multiple sensing electrodes, (ii) a balloon catheter having multiple sensing electrodes disposed on the surface of the balloon, or (iii) a focal catheter having multiple sensing electrodes. Each sensing electrode is configured to produce, in response to sensing electrophysiological (EP) signals in tissue of heart 26, one or more signals indicative of the sensed EP signals.

In some exemplary embodiments, the proximal end of catheter 22 is connected, *inter alia,* to interface circuits 38, so as to transfer these signals to processor 34 for performing the electro-anatomical mapping.

In some exemplary embodiments, during the electro-anatomical mapping, the signals produced by the sensing electrodes of distal-end assembly 40 may comprise thousands of data points, e.g., about 50,000 data points or even more. Based on the data points, processor 34 is configured to present on map 27, vectors indicative of electrical signals propagating over the surface of heart 26, and based on the presented vectors, physician 30 determines one or more sites for ablating tissue in heart 26. However, producing, reviewing and analyzing the aforementioned large number of data points and vectors, may prolong the duration of the ablation procedure. Moreover, the effort associated with producing and analyzing the large amount of data points, may exhaust the physician, and thereby, may damage the quality of the ablation procedure.

In the context of the present disclosure and in the claims, the terms "about" or "approximately" for any numerical values or ranges indicate a suitable dimensional tolerance that allows the part or collection of components to function for its intended purpose as described herein.

In some exemplary embodiments, catheter 22 may comprise one or more ablation electrodes (not shown) coupled to distal-end assembly 40. The ablation electrodes are configured to ablate tissue at a target location of heart 26. After determining the ablation plan, physician 30 navigates distal-end assembly 40 in close proximity to the target location in heart 26, using a manipulator 32 for manipulating catheter 22. Additionally or alternatively, physician 30 may use any other sort of a suitable catheter for ablating tissue of heart, 26 so as to carry out the aforementioned ablation plan.

In some exemplary embodiments, the position of distal-end assembly 40 in the heart cavity is measured using a position sensor (not shown) of a magnetic position tracking system. In the present example, console 24 comprises a driver circuit 41, which is configured to drive magnetic field generators 36 placed at known positions external to patient 28 lying on table 29, e.g., below the patient's torso. The position sensor is coupled to the distal end, and is configured to generate position signals in response to sensed external magnetic fields from field generators 36. The position signals are indicative of the position the distal end of catheter 22 in the coordinate system of the position tracking system.

This method of position sensing is implemented in various medical applications, for example, in the CARTO^{™} system, produced by Biosense Webster Inc. (Irvine, Calif.) and is described in detail in U.S. Patent Nos. 5,391,199, 6, 690, 963, 6,484,118, 6,239,724, 6, 618, 612 and 6, 332, 089, in PCT Patent Publication WO 96/05768, and in U.S. Patent Application Publication Nos. 2002/0065455 A1, 2003/0120150 A1 and 2004/0068178 A1, whose disclosures are all incorporated herein by reference.

In some exemplary embodiments, the coordinate system of the position tracking system are registered with the coordinate systems of system 20 and map 27, so that processor 34 is configured to display on map 27, the position of the distal end of catheter 22.

In some exemplary embodiments, when performing an ablation procedure, physician inserts catheter 22 into a cavity of heart 26, e.g., a left atrium (LA) of the heart. In some cases the procedure may comprise: (i) an anatomical mapping of the LA (not shown), which is carried out by visiting multiple (e.g., thousands) of anatomical points on the surface of the LA tissue. The anatomical mapping is typically followed by an EA mapping of at least a section of interest in the LA. Based on the EA mapping, physician 30 has to perform set up operations that may consume time and, undesirably, may prolong the duration of the procedure. For example, the setup operations may comprise producing a three-dimensional (3D) map of the LA, selection and analysis of EP signals received from catheter 22 during the EA mapping, and producing a suitable graphical user interface (GUI) for performing the procedure.

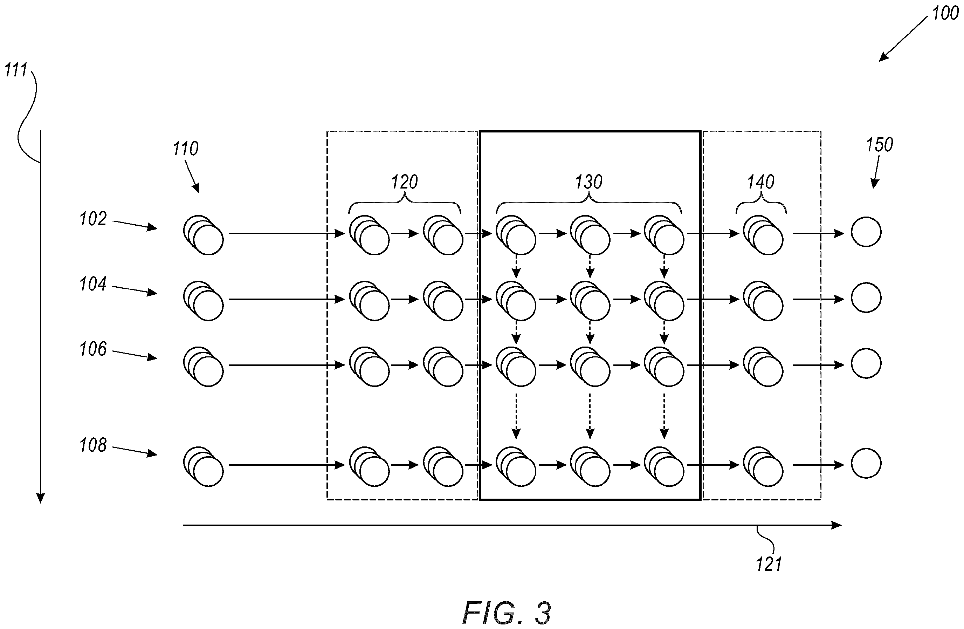

In some exemplary embodiments, processor 34 is configured to perform a partial anatomical mapping of the LA using a small number (e.g., less than about one hundred) anatomical points received from catheter 22. In the present example, processor 34 produces a triangular mesh (not shown) that is based on the anatomical points, and applies to the triangular mesh, a neural network (NN) that is pre-trained. In some embodiments, the NN may comprise a convolutional neural network (CNN), or a recurrent neural network (RNN) or a combination of a CNN and an RNN whose architecture is described, for example, with respect to Fig. 3 below.

In some exemplary embodiments, the CNN may be implemented in processor 34, or in any other processing device of console 24 of system 20, or in any suitable computer external to system 20. The CNN is trained using other anatomical mappings that have been completed and are previously stored in a memory of system 20, or in the memory of the aforementioned external computer. For example, the other anatomical mappings may comprise full anatomical mappings of left atriums of other patients, and/or a full anatomical mapping carried out in a previous procedure performed on the LA of patient 28.

In some exemplary embodiments, processor 34 is configured to receive, based on the data produced during the training of the CNN, a set of templates for each EP procedure applied to each selected cavity (e.g., chamber) of the patient heart. In the present example, processor 34 and/or the CNN may produce or receive one or more GUI templates for respective types of ablation procedures carried out in one or more LAs of respective patient hearts. For example, each GUI template may comprise: (i) setting of a window of interest (e.g., when mapping a ventricle, the window of interest is positioned around the R peak of the electrocardiogram signal(s), and when mapping an atrium, the window of interest is positioned around the P wave of the electrocardiogram signal(s)), (ii) name of the map, and (iii) map view - e.g., anteroposterior (AP) and/or posteroanterior (PA) for viewing the right atrium, and LAO and RAO for a ventricle. Note that the term LAO refers to rotating the view to the patient's left (catheter and spine will be on the right side of the map), and the term RAO refers to the patient's right (catheter and spine on the left side of the map). Additionally or alternatively, the GUI template may comprise any other suitable items.

In some exemplary embodiments, after receiving sufficient anatomical points (e.g., about 1000 points), processor 34 is configured to select from among the GUI templates described above, and based on the partial anatomical mapping, a GUI template that is specified for applying the tissue ablation to the LA of heart 26.

In some exemplary embodiments, processor 34 is configured to present, e.g., on display 35, the selected GUI template to physician 30, for performing the tissue ablation in the LA.

In some cases physician 30 may decide to carry out an additional procedure in another chamber of the patient heart. For example, an EA mapping in the right atrium. In some embodiments, physician 30 inserts the catheter 22 into the right atrium for performing a partial mapping, and processor 34 is configured to select and present a corresponding GUI template to physician 30, using embodiments of the techniques described in detail above, for performing the ablation procedure in the LA of heart 26.

In some exemplary embodiments, processor 34 typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

This particular configuration of system 20 is shown by way of example, in order to illustrate certain problems that are addressed by embodiments of the present invention and to demonstrate the application of these embodiments in enhancing the performance of such a system. Embodiments of the present invention, however, are by no means limited to this specific sort of example system, and the principles described herein may similarly be applied to other sorts of medical systems.

### USING NEURAL-NETWORK BASED GRAPHICAL USER INTERFACE FOR REDUCING SETUP TIME OF ELECTROPHYSIOLOGICAL PROCEDURES

Fig. 2 is a flow chart that schematically illustrates a method for reducing the setup time of an electrophysiological (EP) procedure, using GUI templates and a neural network (NN), in accordance with an embodiment of the present invention. In the present example, the NN comprises a convolutional neural network (CNN), which is described with respect to Fig. 1 above, and the architecture of the CNN is described in detail with respect to Fig. 3 below.

In the exemplary embodiment of Fig. 2, the EP procedure is carried out in a chamber of the heart 26, and may comprise an EA mapping and/or an ablation of tissue in heart 26. In other embodiments, however, the techniques described herein may be used, *mutatis mutandis,* in other suitable sorts of medical procedures.

The method begins at a procedure selection step 50, with physician selecting a medical procedure, such as an EP procedure performed in heart 26, such as but not limited to EA mapping and/or ablation of tissue as described above.

At a catheter insertion step 52, physician 30 inserts catheter 22 into heart 26 for performing the EP procedure in a selected cavity (e.g., chamber) of heart 26 that has given cavity types. In the present example, the left atrium (LA) of heart 26, which is selected for performing the EP procedure, has one or more features different from features of the other chambers of heart 26. Note that the method described herein can be applied, *mutatis mutandis,* to all the chambers of heart 26 (i.e., left ventricle, right atrium and right ventricle).

At a partial anatomical mapping step 54, physician 30 manipulates distal-end assembly 40 for visiting anatomical points on the surface of the LA, so as to acquire mapping points for performing at least a partial anatomical mapping of the LA. In the present example, physician 30 applies fast anatomical mapping (FAM) techniques, and the number of acquired mapping points is substantially smaller compared to the number of mapping points required for a full anatomical mapping. For example, acquisition of about 5000 mapping points are required for full mapping of the LA, whereas in the partial anatomical mapping of step 54, only about 1000 points are acquired.

In some embodiments, in partial anatomical mapping step 54 processor 34 produces a triangular mesh based on the visited and acquired anatomical points. In the context of the present disclosure and in the claims, the term "triangular mesh" refers to a virtual mesh, which is based on the positions of the anatomical points acquired by distal-end assembly 40 during the partial anatomical mapping of step 54. The mesh is made from virtual triangles, such that the acquired anatomical points are positioned on respective vertices of the triangles of the mesh. Processor 34 is configured to define the structure (topology and geometry) of the virtual mesh (e.g., the size and direction of the triangles), based on the anatomical points acquired in partial anatomical mapping step 54. In such embodiments, step 54 outputs the virtual mesh of triangles having the acquired anatomical points positioned on respective vertices of the mesh.

At a first decision step 56, processor 34 checks whether the number of mapping points acquired in step 54 is sufficient for proceeding in the method. If not, the method loops back to step 54 until a sufficient number of mapping points (e.g., larger than about 1000 points) have been acquired. Note that processor 34 is configured to alter the topology and/or geometry of the mesh based on additional mapping points that have been acquired by distal-end assembly 40.

In case the number of mapping points acquired in step 54 is sufficient, the method proceeds to a chamber verification step 58, in which the CNN (which may be implemented in processor 34 or in any other device of system 20) identifies, based on the partial anatomical mapping, that the acquired mapping points have features unique to the LA of a heart of other patients. For example, a shape of a pulmonary vein (PV) that transfers blood from the lungs to heart 26 of patient 28.

At a GUI template selection step 60, processor 34 selects, based on the partial anatomical mapping carried out in step 54 above, and the chamber identification in step 58, a GUI template that is specified for applying the EP procedure to the LA of heart 26.

In some embodiments, the GUI template is selected by processor 34 from among multiple GUI templates that are produced before the procedures, and based on the chamber identified by the CNN, which is trained before conducting the EP procedure. In the present example, the CNN is pre-trained using other anatomical mappings, such as but not limited to full anatomical mappings of heart chambers, which are previously stored in any suitable memory device of system 20 and/or in processor 34. In such embodiments, the CNN is trained on the stored anatomical maps, and after concluding the training, the CNN is applied to the mesh of triangles produced during the partial anatomical mapping described in step 54 above, so as to identify the chamber (e.g., LA) of heart 26.

In some embodiments, based on the identified chamber and the selected procedure, processor 34 is configured to select, from among the GUI templates produced before the procedure, a GUI template suitable for performing the selected EP procedure in the LA of heart 26, as described above.

In one implementation, the training of the neural network for identifying the chamber based on the partial anatomical mapping, may be carried out using a random walk technique over the topological and geometrical presentation of respective meshes produced by respective stored anatomical maps. Subsequently, the representation of the random walk may be prepared and inserted into a recurrent neural network (RNN) that accumulates the properties of the random walk for updating the neural network. The trained neural network is applied to the mesh of triangles produced during the partial anatomical mapping and may automatically output the identified chamber (e.g., LA) of heart 26, so that processor 34 can select the most suitable GUI template from among the GUI templates produced before the procedure (e.g., by processor 34 and/or by the neural network, or by any other suitable device). In some cases the RNN may be combined with a CNN so as to an architecture that may obtain improved performance of the trained neural network. The automatic identification of the LA is described in detail in a paper titled "MeshWalker: Deep Mesh Understanding by Random Walks" by Lahav et al., ACM Trans. Graph., Vol. 39, No. 6, Article 263, December 2020, whose disclosure is incorporated herein by reference.

Note that steps 58 and 60 may be combined into a single step, so that (i) the trained neural network identifies the chamber based on the partial anatomical mapping, and (ii) based on the chamber identification and selected procedure, processor 34 selects the most suitable GUI template. In the present example, the selected GUI template is specified for applying the selected EP procedure (e.g., tissue ablation) to the LA of heart 26.

At a GUI-template presentation step 62, processor 34 presents the selected GUI template and a map of the left atrium to a user, for performing the EP procedure in the LA. At a second decision step 64, processor 34 checks whether or not the EA mapping and/or the tissue ablation procedure have been completed. In case the mapping and/or ablation procedure have been completed, the method ends, and physician may extract distal-end assembly 40 out of the LA. In case the EA mapping and/or the tissue ablation procedure have been completed, the method proceeds to a procedure continuation step 66 in which physician 30 continues the procedure.

At a third decision step 68, processor 34 checks whether physician 30 decides to change the treated chamber and/or the type of procedure. In an embodiment, physician may decide to perform an EA mapping to the right atrium (RA) of heart 26. In this embodiment, the method loops back to step 50 for registering the aforementioned selection in processor 34, and the method repeats for performing the EA mapping in the RA of heart 26.

In case physician 30 retains the selected EP procedure carried out in the LA, the method loops back from step 68 to step 66, for continuing and concluding the originally-selected EP procedure carried out in the LA of heart 26, as described above.

Fig. 3 is a schematic block diagram illustrating an architecture of a neural network (NN) 100 applied to partial anatomical mapping of a heart cavity for reducing the setup time of an EP procedure, in accordance with an embodiment of the present invention. In the present example, the heart cavity comprises the left atrium (LA) and the EP procedure comprises electro-anatomical (EA) mapping of the LA or ablation of tissue in the LA of heart 26, as described in detail in the method of Fig. 2 above.

In some embodiments, the architecture of NN 100 comprises a convolutional neural network (CNN) having layers of a recurrent neural network (RNN), also referred to herein as RNN layers 130 that are described in more detail below.

In some embodiments, an arrow 111 represents a sequence of steps (e.g., steps 102, 104, 106 and 108) of the aforementioned random walk, which is applied to the mesh of triangles (formed in the partial anatomical mapping described in step 54 of Fig. 2 above) in a process of identifying the heart chamber (e.g., LA) by NN 100.

In some embodiments, an arrow 121 is indicative of the depth of NN 100. In the present example, NN 100 comprises layers 110, 120, 130, 140 and 150 described herein, but in other embodiments, NN 100 may comprise any suitable additional or alternative layers.

In some embodiments, an input layer 110 provides the position in the mesh of triangles of the random walk described in Fig. 2 above. In the present example, layer 110 has a three-dimensional (3D) output.

In some embodiments, at step 102 the random walk begins over the mesh of triangles, and the length of the walking path increases gradually in steps 104, 106 and 108.

In some embodiments, layer 120 comprises a fully-connected (FC) layer that operates on a flattened input (e.g., converting the input data into a one-dimensional array), so that each input is connected to all the available neurons. In the present example, layer 120 comprises one or more FC layers, and is configured to produce an output dimension of 256.

In some embodiments, RNN layers 130 comprise one or more layers, and is configured to remember and accumulate the random walk carried out in steps 102-106 based on the input of layer 110 and FC layer(s) 120. In the present example, RNN layers 130 comprise three layers and has an output dimension of 512. In RNN layers 130, connections between nodes form a directed graph along a temporal sequence. Moreover, RNN layers 130 can use their internal state (memory) to process variable length sequences of inputs provided in layer 110 by steps 102-108.

In some embodiments, RNN layers 130 are configured to remember and accumulate knowledge along the sequence of steps 102-108, and are not confined to a fixed length of inputs or outputs. The output of RNN layers 130 comprises a state vector that contains the information gathered along the walk provided by the input of layer 110.

In some embodiments, layer 140 comprises a FC layer configured to output a number of classes. In the present example, layer 140 may output four classes corresponding to the four chambers of heart 26.

In some embodiments, layer 140 comprises a prediction layer, which is configured to output, based on the knowledge accumulate along the sequence of steps 102-108, a single descriptor vector. In the present example, the vector comprises the identified chamber, e.g., the left atrium of heart 26. Note that, together, layers 140 and 150 provide the classification of an object whose shape is provided by the mesh of triangles, in the present example, the LA selected from among the cavities or chambers of heart 26.

In some embodiments, the output of layer 150 in steps 102 and 104 is typically meaningless because the information gathered during the walk is insufficient. The output of layer 150 in step 106 can be one of the chambers of heart 26, and the output of layer 150 in step 108 is the LA of heart 26, which is identified based on the accumulated information gathered during the walk in the mesh of triangles described above.

As described in step 58 of Fig. 2 above, and further described in the architecture and operational steps of NN 100, processor 34 applies the neural network (e.g., NN 100 or any other suitable architecture of NN) so as to identify the LA based on the partial anatomical mapping.

In some embodiments, after NN 100 identifies the LA based on the partial anatomical mapping, processor 34 may select and display (e.g., on display 35) a selected GUI template, as described in respective steps 60 and 62 of Fig. 2 above. Note that the GUI template is selected from among multiple GUI templates prepared in advance, and is specified for applying the medical procedure (e.g., tissue ablation) to the corresponding chamber (e.g., the left atrium) of heart 26.

This particular architecture of NN 100 is provided with reference to the aforementioned paper of Lahav et al. that describes the NN architecture in more detail, and whose disclosure is incorporated herein by reference. However, NN 100 is shown by way of example, in order to illustrate certain problems that are addressed by embodiments of the present invention and to demonstrate the application of these embodiments in enhancing the performance of system 20. Embodiments of the present invention, however, are by no means limited to this specific sort of example neural network, and the principles described herein may similarly be applied, *mutatis mutandis,* to other sorts of suitable types of neural networks as well as other suitable architectures of CNNs that may or may not be combined with RNNs.

Although the embodiments described herein mainly address electrophysiological (EP) procedures, such as electro-anatomical mapping and tissue ablation performed in patient heart, the methods and systems described herein can also be used in other applications, such as in structural heart procedures, and in any EP procedure carried out on an organ of a patient, other than heart.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A method for reducing the setup time for electrophysiological procedures, comprising:
inserting a catheter into a cavity of a patient organ, for performing a medical procedure, wherein the cavity is of a given cavity type;
performing a partial anatomical mapping of the cavity by visiting, using the catheter, one or more anatomical points on a surface of the cavity;
selecting, based on the partial anatomical mapping, a Graphical User Interface (GUI) template that is specified for applying the medical procedure to the given cavity type; and
presenting the selected GUI template to a user for performing the medical procedure in the cavity.

2. The method according to claim 1, wherein selecting the GUI template comprises choosing the GUI templates from among multiple GUI templates specified for multiple respective cavity types.

3. The method according to claim 1, wherein selecting the GUI template comprises applying, to the partial anatomical mapping, a neural network that is pre-trained using one or more other anatomical mappings of one or more cavities.

4. The method according to claim 3, wherein performing the partial anatomical mapping comprises producing a triangular mesh of the visited anatomical points, and wherein selecting the GUI template comprises applying the pre-trained neural network to the triangular mesh

5. The method according to claim 1, wherein selecting the GUI template comprises identifying the given cavity type based on the partial anatomical mapping, and selecting the GUI template based on the identified cavity type. the identified cavity type and on a type of the medical procedure.

6. A system for reducing the setup time for electrophysiological procedures, comprising:
a processor, which is configured to: (i) receive, from a catheter inserted into a patient organ having a cavity of a given cavity type and visiting one or more anatomical points on a surface of the cavity, a partial anatomical mapping of the cavity, and (ii) select, based the partial anatomical mapping, a Graphical User Interface (GUI) template that is specified for applying the medical procedure to the given cavity type; and
an output device, which is configured to present the selected GUI template to a user for performing the medical procedure in the cavity.

7. The system according to claim 6, wherein the processor is configured to select the GUI templates from among multiple GUI templates specified for multiple respective cavity types.

8. The system according to claim 6, wherein the processor is configured to apply to the partial anatomical mapping, a neural network that is pre-trained using one or more other anatomical mappings of one or more cavities.

9. The system according to claim 8, wherein the processor is configured to: (i) perform the partial anatomical mapping by producing a triangular mesh of the visited anatomical points, and (ii) select the GUI template by applying the pre-trained neural network to the triangular mesh.

10. The method according to claim 3 or the system according to claim 8, wherein a first number of the visited anatomical points in the partial anatomical mapping is smaller than a second number of anatomical points used for training the neural network.

11. The method according to claim 3 or the system according to claim 8, wherein at least one of the one or more cavities is mapped in another patient.

12. The method according to claim 1 or the system according to claim 6, wherein the patient organ comprises a patient heart, and wherein the medical procedure comprises an ablation of tissue of the patient heart.

13. The method according to claim 1 or the system according to claim 6, wherein the patient organ comprises a patient heart, and wherein the medical procedure comprises an electro-anatomical mapping of tissue of the patient heart.

14. The method according to claim 13, wherein selecting the GUI template comprises selecting the GUI template based on

15. The system according to claim 6, wherein the processor is configured to identify the given cavity type based on the partial anatomical mapping, and to select the GUI template based on the identified cavity type, optionally wherein the processor is configured to select the GUI template based on the identified cavity type and on a type of the medical procedure.
